# EUROPÄISCHE PATENTSCHRIFT

(11) **EP 1 328 520 B1**
(45) Veröffentlichungstag und Bekanntmachung des Hinweises auf die Patenterteilung: **05.12.2007**
(21) Anmeldenummer: 01978413.1
(22) Anmeldetag: 15.10.2001
(51) Int. Cl.: C07D 251/60

(54) **VERFAHREN ZUR HERSTELLUNG VON MELAMIN**
METHOD FOR PRODUCING MELAMINE
PROCEDE DE FABRICATION DE MELANINE

(30) Priorität: 20.10.2000 AT 18022000
(43) Veröffentlichungstag der Anmeldung: 23.07.2003
(73) Patentinhaber: AMI Agrolinz Melamine International GmbH, 4020 Linz (AT)
(72) Erfinder: BUCKA, Hartmut, A-4622 Eggendorf 125 (AT); COUFAL, Gerhard, A-4060 Leonding (AT); KOGLGRUBER, Ferdinand, A-4040 Linz (AT)
(74) Vertreter: Gross, Felix
(86) Internationale Anmeldenummer: PCT/EP2001/011890
(87) Internationale Veröffentlichungsnummer: WO 2002/034730

(56) Entgegenhaltungen:
- WO-A-99/00374
- DE-B- 1 228 266

## Beschreibung

Die Erfindung betrifft ein Verfahren und eine Vorrichtung zur Herstellung von Melamin durch Pyrolyse von Harnstoff.

Bei den Hochdruckverfahren zur Herstellung von Melamin wird Harnstoff über eine endotherme Flüssigphasenreaktion zu Melamin umgesetzt. Das flüssige Melamin enthält je nach den Druck- und Temperaturbedingungen im Reaktor zusätzlich unterschiedliche Mengen an gelöstem NH₃ und CO₂ sowie Kondensationsnebenprodukte und nicht umgesetzten Harnstoff. Das so erhaltene Melamin wird anschließend etwa durch Quenchen mit Wasser oder mit Ammoniak, durch Sublimation mit nachfolgender Desublimation oder durch Entspannen unter bestimmten Bedingungen verfestigt.

Als Reaktor dient üblicherweise ein Tankreaktor mit Zentralrohr und außerhalb des Zentralrohres angeordneten Heizelementen, die die zur Reaktion nötige Wärme bereitstellen. Diese Heizelemente sind parallel zum Zentralrohr angeordnete Rohrbündel, in denen eine Salzschmelze zirkuliert. Dabei werden Harnstoff und NH₃ am Boden des Reaktors eingebracht, treffen auf eine Verteilerplatte, die sich unterhalb des Zentralrohres befindet und reagieren im freien Raum zwischen den Rohrbündeln, in dem sich bereits Melamin befindet, unter Zersetzung und Gasentwicklung zu Melamin. In WO99/00374 ist ein solcher Reaktor schematisch abgebildet, wobei auch die Strömungsrichtung der Schmelze so angegeben ist, dass das Reaktionsgemisch außerhalb des Zentralrohres zwischen den Rohrbündeln nach oben strömt und sich dort in Offgas und flüssiges Melamin trennt. Das Offgas wird am Kopf des Reaktors abgezogen, ein Teil der Melaminschmelze wird über einen Überlauf aus dem Reaktor ausgetragen und der andere Teil der Melaminschmelze fließt innerhalb des Zentralrohres aufgrund der Schwerkraft nach unten.

Dieser bisher verwendete Reaktortyp hat jedoch den Nachteil, daß die Rohrbündel insbesondere bei höherem Harnstoffdurchsatz relativ rasch korrodieren und daher häufig ausgewechselt werden müssen.

Unerwarteterweise wurde nun gefunden, daß die Korrosionsrate der Salzschmelze-Rohre wesentlich gesenkt werden kann, wenn die Vermischung des Harnstoffes mit Melamin und seine Zersetzung nicht außerhalb, sondern innerhalb des Zentralrohres erfolgt. Entgegen der ursprünglichen Annahme, dass die Strömungsrichtung der Melaminschmelze so ist, wie in WO99/00374 angegeben, wurde darüber hinaus gefunden, dass die Strömungsrichtung der Melaminschmelze bei der erfindungsgemäßen Anordnung genau umgekehrt ist, die Schmelze strömt nämlich innerhalb des Zentralrohres nach oben und außerhalb des Zentralrohres nach unten.

Die für die insgesamt endotherme Reaktion nötige Wärmezufuhr erfolgt durch die außerhalb des Zentralrohres angeordneten Heizrohre bei der Bewegung der Schmelze nach unten, sodass im unteren Teil des Reaktors eine um etwa 3 - 30 °, bevorzugt um 5 - 15 °C höhere Temperatur herrscht, als im oberen Teil. Dass die Melaminschmelze im oberen Teil des Reaktors, wo sie über eine Überlauf abgezogen wird, kälter ist, als im unteren Teil bedeutet einen weiteren Vorteil gegenüber der Anordnung nach WO99/00374, da die Melaminschmelze in den nachfolgenden Schritten weniger gekühlt werden muss, und die Gleichgewichtslage der Schmelze bei niedrigerer Temperatur in Richtung Melamin verschoben ist, sodass weniger Nebenprodukte gebildet werden.

Gegenstand der Erfindung ist demnach ein Verfahren zur Herstellung von Melamin durch Pyrolyse von Harnstoff in einem Hochdruckreaktor mit einem senkrechten Zentralrohr unter Bildung einer Melaminschmelze, das dadurch gekennzeichnet ist, dass
- die im Reaktor zirkulierende Melaminschmelze sich im unteren Bereich des Reaktors mit einer von unten in den Reaktor eingebrachten Harnstoffschmelze und gegebenenfalls eingebrachtem NH₃ vermischt.
- die gebildete Reaktionsmischung, bestehend im wesentlichen aus Melamin, NH₃, CO₂ und gegebenenfalls Reaktionszwischenprodukten im Zentralrohr von unten nach oben strömt,
- die gebildete Reaktionsmischung im oberen Teil des Zentralrohres aus dem Zentralrohr austritt,
- am Reaktorkopf oberhalb des Zentralrohres die Auftrennung zwischen Melamin und Offgas stattfindet,
- ein Teil des oben aus dem Zentralrohr austretenden Melamins im Ringraum zwischen Zentralrohr und Reaktorwand nach unten strömt und der restliche Teil zur weiteren Aufarbeitung ausgeschleust wird
- die Offgase am Reaktorkopf ausgeschleust werden.

Zur Durchführung des erfindungsgemäßen Verfahrens wird Harnstoff, der bevorzugt als ammoniakgesättigte Harnstoffschmelze aus einem Harnstoffwäscher kommt, mit einer Temperatur von etwa 135 - 250°C, von unten in den Melaminreaktor eingebracht. Gemeinsam mit dem Harnstoff wird gegebenenfalls NH₃ von unten in den Reaktor eingetragen. Dabei beträgt das Molverhältnis von dem dem Melaminreaktor gegebenenfalls zugeführten NH₃ zum zugeführten Harnstoff etwa 0-10 mol, bevorzugt etwa 0-5 mol, besonders bevorzugt etwa 0-2 mol NH₃ / mol Harnstoff. Der Druck im Melaminreaktor liegt je nach gewähltem Temperaturbereich in einem Bereich von etwa 50 - 350 bar, bevorzugt von etwa 80 - 250 bar.

Die Temperatur im Melaminreaktor liegt je nach gewähltem Druckbereich in einem Bereich von etwa 320 - 450°C, bevorzugt von etwa 320 - 400°C, besonders bevorzugt von etwa 330 - 380 °C.

Der Melaminreaktor ist ein Tankreaktor mit senkrecht stehendem Zentralrohr. Die von unten in das Zentralrohr eingebrachte Harnstoffschmelze und das gegebenenfalls eingebrachte NH₃ strömen bevorzugt gegen eine im unteren Teil des Zentralrohres angebrachte Verteilerplatte und dann weiter entweder an der Verteilerplatte vorbei oder durch Öffnungen bzw. Düsen, die in einer Haltevorrichtung, beispielsweise einem Halteblech zur Befestigung der Verteilerplatte, am Einleitrohr für Harnstoff und NH₃ angeordnet sind, durch die Verteilerplatte hindurch in Richtung Zentralrohr. Die Reaktanten vermischen sich im Inneren des Zentralrohres mit der ebenfalls von unten in das Zentralrohr einströmenden, im Reaktor zirkulierenden Melaminschmelze.

Durch die intensive Vermischung der kühlen Harnstoffschmelze mit der heissen, zirkulierenden Melaminschmelze im Zentralrohr kommt es zur Erwärmung der Reaktanten, und der Harnstoff pyrolysiert über die Reaktorhöhe zu Melamin und Offgas, hauptsächlich bestehend aus NH₃ und CO₂. Da die Melaminbildung endotherm ist, muss die Menge des im Reaktor zirkulierenden Melamins so groß sein, daß durch die Temperaturerniedrigung des Melamins beim Vermischen der Reaktanten und während der Harnstoffpyrolyse nicht die Gefahr der Melaminverfestigung besteht.

Die Einstellung des im Reaktor gewünschten Temperaturprofiles kann durch die eingebrachte Harnstoffmenge, die Temperatur der Salzschmelze und die Zirkulationsrichtung der Salzschmelze in den Doppelmantelrohren erfolgen.

Weiters ist es möglich, am Reaktorboden oder im Zentralrohr selbst Einbauten, Verteilerböden oder Strömungsleitbleche oder ähnliches anzubringen, die eine Vergleichmäßigung der Strömung bei der Umleitung der Melaminschmelze vom Ringraum in das Zentralrohr, eine bessere Verteilung der Schmelzeströme und die Vergleichmäßigung der Blasen innerhalb des Zentralrohres, sowie eine bessere Auftrennung zwischen Melaminschmelze und Offgas beim Austritt aus dem Zentralrohr und am Reaktorkopf ermöglichen.

Im oberen Reaktorteil erfolgt die Auftrennung zwischen Offgas und flüssigem Melamin. Die Melaminschmelze kann dort sowohl am oberen Ende des Zentralrohres als auch zusätzlich durch seitliche Öffnungen im Zentralrohr in den ringförmigen Raum zwischen Zentralrohr und Reaktorinnenwand austreten.

Ein Teil des Melamins strömt in diesem Ringraum nach unten, während die restliche Melaminschmelze zur weiteren Aufarbeitung über einen Überlauf aus dem Reaktor ausgeschleust wird. Die Offgase werden kontinuierlich am Kopf des Reaktors bevorzugt in Richtung Harnstoffwäscher abgezogen. Vorteilhafterweise sind im Bereich der Auftrennung zwischen Offgas und flüssigem Melamin Prallplatten oder Gitter als Beruhigungszone und zur Verbesserung der Trennwirkung angeordnet.

Im ringförmigen Bereich zwischen dem Zentralrohr und der Reaktorwand befinden sich zumeist vertikale Heizrohre, mit deren Hilfe dem Reaktor die für die endotherme Reaktion nötige Wärmemenge zugeführt wird. Ein Teil der aus dem Zentralrohr überlaufenden Melaminschmelze bewegt sich im Ringraum aufgrund der höheren Dichte nach unten, vermischt sich im unteren Zentralrohrbereich erneut mit eingebrachtem Harnstoff und bewirkt somit eine interne Zirkulation im Reaktor.

Das restliche, über einen Überlauf am Kopf des Reaktors kontinuierlich ausgetragene Melamin wird in beliebiger Weise aufgearbeitet und verfestigt. Dies kann beispielsweise durch Entspannen des mit Ammoniak gesättigten Melamins bei einer Temperatur, die knapp über ihrem druckabhängigen Schmelzpunkt liegt, durch Verfestigen in einer Wirbelschicht oder durch Quenchen mit Wasser, mit flüssigem oder gasförmigem Ammoniak oder durch Sublimieren und anschließendes Desublimieren aus der Gasphase erfolgen.

Ein weiterer Gegenstand der Erfindung ist ein Reaktor zur Herstellung von Melamin durch Pyrolyse von Harnstoff, bestehend aus einem senkrecht stehenden Reaktorkörper mit Zentralrohr, im unteren Teil des Reaktors angebrachten Zuleitungen für Harnstoff und gegebenenfalls NH₃, im oberen Teil des Reaktors angebrachten Ableitungen für das gebildete Melamin und für die im wesentlichen aus NH₃ und CO₂ bestehenden Offgase, Heizeinrichtungen und Meß- und Regeleinrichtungen, insbesondere für Temperatur, Druck, Durchflußmengen und Standhöhe der Schmelze, dadurch gekennzeichnet, daß eine oder mehrere Austrittsöffnungen für die Zuleitung von Harnstoffschmelze und gegebenenfalls NH₃ innerhalb des Zentralrohres angeordnet sind.

Im unteren Bereich des Zentralrohres ist bevorzugt eine Verteilerplatte zur Verteilung des einströmenden Harnstoffs und des gegebenenfalls eingebrachten NH₃ angebracht. Die Verteilerplatte kann entweder als ebene Platte ausgebildet sein, oder aber zur besseren Verteilung des nach oben strömenden Harnstoffstromes und des ebenfalls nach oben strömenden Melaminstromes beliebige geometrische Formen aufweisen wie z.B. die Form einer Pyramide, einer Halbschale oder bevorzugt die Form eines Kegels.

Es ist besonders vorteilhaft, wenn der Spalt zwischen der Verteilerplatte (3) und der Austrittsöffnung des Einleitrohres für die Harnstoffschmelze und gegebenenfalls NH₃ möglichst klein ist, etwa ein Ringspalt von 3 - 13 mm Querschnitt oder Öffnungen bzw. Düsen, die in einer Haltevorrichtung, beispielsweise einem Halteblech zur Befestigung der Verteilerplatte am Einleitrohr für Harnstoff und gegebenenfalls NH₃ angeordnet sind. Die Öffnungen oder Düsen können eine beliebige geometrische Form aufweisen und sind beispielsweise kreisförmig, ringförmig oder in Form eines Ringspaltes ausgeführt. Die Öffnungen oder Düsen sind so dimensioniert, daß die Austrittsgeschwindigkeit an den Öffnungen bzw. Düsen 0,2 - 10 m/sec., bevorzugt 1 - 5 m/sec., besonders bevorzugt 0,5 - 1 m/sec. beträgt und dadurch die Reaktanten im Melamin fein zerteilt werden. Durch diese Anordnung wird eine höhere Austrittsgeschwindigkeit für Harnstoff und gegebenenfalls NH₃ erreicht, welche eine bessere, intensivere und noch homogenere Durchmischung der Reaktanten mit der aufwärts strömenden Melaminschmelze ermöglicht. Nach dem Austreten aus dem Einleitrohr wird der Harnstoffstrom bevorzugt in Richtung Zentralrohr umgelenkt, so daß er in gleicher Strömungsrichtung mit dem Melamin fließt.

Das Einströmen der im Reaktor zirkulierenden Melaminschmelze aus dem ringförmigen Raum zwischen Reaktorwand und Zentralrohr in die Mischzone mit der Harnstoffschmelze und dem gegebenenfalls eingebrachten NH₃ kann beispielsweise durch seitliche Öffnungen im unteren Bereich des Zentralrohres ermöglicht werden.

Eine mögliche Ausführungsform des Reaktors mit ebener Verteilerplatte im Zentralrohr ist schematisch in Fig. 1 dargestellt. Fig. 2 enthält eine bevorzugte Ausführung der Verteilerplatte in Form eines Kegels und den Einbau von Strömungsleitblechen. Fig. 3 zeigt im oberen Teil den Eintrag der Harnstoffschmelze mittels Düsen in das Innere des Zentralrohres, und im unteren Teil einen Querschnitt, Fig. 4 zeigt den Harnstoffeintrag über Ringspalte im Querschnitt.

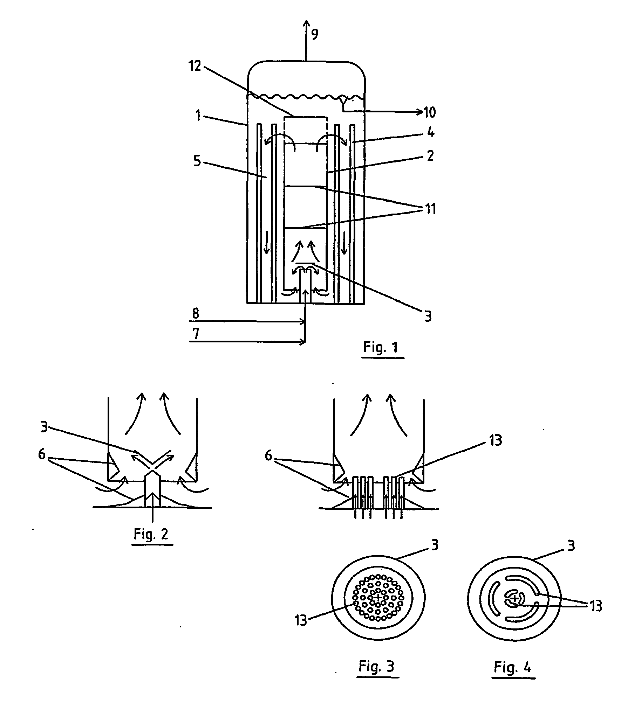

Fig. 1 bis Fig. 4 zeigen: (1) Melaminreaktor, (2) Zentralrohr, (3) Verteilerplatte, (4) Heizrohre, (5) ringförmiger Raum, (6) Strömungsleitbleche, (7) zugeführte Harnstoffschmelze, (8) NH₃ Gas, (9) Offgase, (10) Melaminschmelze zur weiteren Aufarbeitung, (11) Einbauten, (12) Prallblech, (13) Düsen- oder Ringspalt.

Der Reaktor besteht aus korrosionsbeständigem Material oder ist mit korrosionsbeständigem Material ausgekleidet, beispielsweise mit Titan.

Es ist möglich, am Reaktorboden, im Zentralrohr und / oder in der Trennzone am Reaktorkopf Einbauten, Verteilerböden, Strömungsleitbleche oder ähnliches anzubringen, die eine Vergleichmäßigung der Strömung bei der Umlenkung der Melaminschmelze vom Ringraum in das Zentralrohr, eine bessere Vermischung von Harnstoff- und Melaminschmelze, eine Vergleichmäßigung der Blasengröße innerhalb des Zentralrohres und beim Austritt aus dem Zentralrohr sowie eine bessere Auftrennung zwischen Melaminschmelze und Offgas am Reaktorkopf ermöglichen.

Die vertikalen Heizrohre (4), durch welche die für die Reaktion nötige Wärme bereitgestellt wird, sind bevorzugt Doppelmantelrohre, in denen eine Salzschmelze zirkuliert. Dabei kann der Zulauf der Salzschmelze entweder über den inneren Rohrquerschnitt und der Ablauf über den äußeren Rohrmantel oder in umgekehrter Fließrichtung erfolgen.

Durch die Vermischung und Reaktion der Reaktanten innerhalb des Zentralrohres kommt ihre korrodierende Wirkung in viel geringerem Maße zum Tragen. Beispielsweise beträgt in einem Melaminreaktor gemäß vorliegender Erfindung, wie in Fig.1 schematisch dargestellt, bei einer Leistung von 2,5 t Melamin/h die Verminderung der Rohrwanddicke jener Heizrohre (4), die dem Zentralrohr (2) am nächsten liegen, etwa 0,1 mm/Jahr. Im Vergleich dazu beträgt die Verminderung der Rohrwanddicke bei gleich hohem Durchsatz, jedoch bei Vermischung der Reaktanten außerhalb des Zentralrohres (2) bis zu etwa 0,9 mm/Jahr.

Die im Reaktor zirkulierende Melaminschmelze dient als Wärmeüberträgermedium für die in den Reaktor eingebrachte Harnstoffschmelze. Dabei stellt sich entsprechend der fortschreitenden Harnstoffpyrolysereaktion über die Zentralrohrhöhe ein insgesamt abfallendes Temperaturprofil ein, d.h. in der Nähe des Melaminüberlaufs aus dem Reaktor herrscht eine niedrigere Temperatur als am Reaktorboden. Daher ist die Melaminaustrittstemperatur aus dem Reaktor niedriger als bei den meisten Melaminverfahren, bevorzugt liegt sie zwischen 330 und 380 °C, besonders bevorzugt zwischen 340 und 370 °C. Ein besonderer Vorteil der umgekehrten Strömungsrichtung ist die niedrige Austrittstemperatur der Melaminschmelze aus dem Synthesereaktor, die nur so niedriger als bei den bisher bekannten Verfahren gefahren werden kann. Der Melaminsynthesereaktor wirkt im Oberteil als Vorkühler. Damit gelangt die vorgekühlte, von den Offgasen getrennte Melaminschmelze schon von Anfang an mit einem geringeren Nebenproduktanteil zu den nächsten Aufarbeitungsschritten.

Darüber hinaus wird durch die reine Flüssigkeitsströmung - zum Unterschied einer Zweiphasenströmung bei umgekehrter Zirkulationsrichtung der Melaminschmelze - eine Verringerung des Druckverlustes im Ringraum zwischen den Salzschmelzerohren und somit eine Erhöhung der Zirkulationsmenge im Reaktor erreicht. Dadurch verbessert sich der Wärmeübergang der Salzschmelze auf die Melaminschmelze.

Weiters besteht die Möglichkeit, durch Einbauten im Zentralrohr die Strömung sowie die Blasengröße und -verteilung des aufwärts strömenden Reaktionsgemisches zu beeinflussen, wodurch eine weitere Verbesserung des Stoff- und Wärmeüberganges erreicht werden kann.

## Patentansprüche

1. Verfahren zur Herstellung von Melamin durch Pyrolyse von Harnstoff in einem Hochdruckreaktor mit einem senkrechten Zentralrohr unter Bildung einer Melaminschmelze, **dadurch gekennzeichnet, dass**
- die im Reaktor zirkulierende Melaminschmelze sich im unteren Bereich des Reaktors mit einer von unten in den Reaktor eingebrachten Harnstoffschmelze und gegebenenfalls eingebrachtem NH₃ vermischt
- die gebildete Reaktionsmischung, bestehend im wesentlichen aus Melamin, NH₃, CO₂ und gegebenenfalls Reaktionszwischenprodukten, im Zentralrohr von unten nach oben strömt
- die gebildete Reaktionsmischung im oberen Teil des Zentralrohres aus dem Zentralrohr austritt
- am Reaktorkopf oberhalb des Zentralrohres die Auftrennung zwischen Melamin und Offgas stattfindet
- ein Teil des oben aus dem Zentralrohr austretenden Melamins im Ringraum zwischen Zentralrohr und Reaktorwand nach unten strömt und der restliche Teil zur weiteren Aufarbeitung ausgeschleust wird
- die Offgase kontinuierlich am Reaktorkopf ausgeschleust werden.

2. Verfahren gemäß Anspruch 1, **dadurch gekennzeichnet, dass** die Harnstoffschmelze und gegebenenfalls das NH₃ von unten in das Zentralrohr eingebracht werden.

3. Verfahren gemäß Anspruch 1, **dadurch gekennzeichnet, dass** die Temperatur der Melaminschmelze am oberen Ende des Zentralrohres niedriger ist als am unteren Ende.

4. Verfahren gemäß Anspruch 1, **dadurch gekennzeichnet, daß** die in das Zentralrohr eingebrachte Harnstoffschmelze und gegebenenfalls NH₃ im unteren Teil des Zentralrohres gegen eine Verteilerplatte strömen.

5. Verfahren gemäß Anspruch 1, **dadurch gekennzeichnet, dass** die im Reaktor zirkulierende Melaminschmelze durch seitliche, im unteren Teil des Zentralrohres angeordnete Öffnungen in das Zentralrohr eintritt, im Zentralrohr nach oben und im Ringraum zwischen Zentralrohr und Reaktorwand nach unten strömt.

6. Reaktor zur Herstellung von Melamin durch Pyrolyse von Harnstoff, bestehend aus einem senkrecht stehenden Reaktorkörper mit Zentralrohr, im unteren Teil des Reaktors angebrachten Zuleitungen für Harnstoffschmelze und gegebenenfalls NH₃, im oberen Teil des Reaktors angebrachten Ableitungen für die gebildete Melaminschmelze und für die im wesentlichen aus NH₃ und CO₂ bestehenden Offgase, Heizeinrichtungen und gegebenenfalls Meß- und Regeleinrichtungen, insbesondere für Temperatur, Druck, Durchflussmengen und Standhöhe der Melaminschmelze, **dadurch gekennzeichnet, dass** eine oder mehrere Austrittsöffnungen für die Zuleitung von Harnstoffschmelze und gegebenenfalls NH₃ im unteren Teil und innerhalb des Zentralrohres angeordnet sind.

7. Reaktor gemäß Anspruch 6, **dadurch gekennzeichnet, dass** im unteren Teil des Zentralrohres, oberhalb der Zuleitungen für Harnstoff und gegebenenfalls NH₃, eine Verteilerplatte angebracht ist.

8. Reaktor gemäß Anspruch 7, **dadurch gekennzeichnet, dass** die Verteilerplatte mit einer oder mehrerer Öffnungen in Richtung Zentralrohr zum Durchgang der Harnstoffschmelze und gegebenenfalls NH₃ versehen ist.

9. Reaktor gemäß Anspruch 7, **dadurch gekennzeichnet, dass** die Verteilerplatte die Form einer ebenen Platte, eines Kegels, einer Pyramide oder einer Halbschale aufweist.

10. Reaktor gemäß Anspruch 6, **dadurch gekennzeichnet, dass** sich im Zentralrohr Einbauten zur Vergleichmäßigung der Strömung und der Zerkleinerung der Gasblasen sowie zur Verbesserung der Durchmischung befinden.

11. Reaktor gemäß Anspruch 6, **dadurch gekennzeichnet, dass** sich am Reaktorkopf oberhalb des Zentralrohres in der Trennzone eine Prallplatte und darüber eine Beruhigungszone befindet.

12. Reaktor gemäß Anspruch 6, **dadurch gekennzeichnet, dass** das Zentralrohr in seinem unteren und/oder oberen Teil seitliche Öffnungen aufweist.

## Claims

1. Process for the production of melamine by pyrolysis of urea in a high-pressure reactor having a vertical central pipe with formation of a melamine melt, **characterized in that**
- the melamine melt circulating in the reactor mixes in the lower region of the reactor with a urea melt introduced into the reactor from below and optionally introduced NH₃,
- the reaction mixture formed, consisting essentially of melamine, NH₃, CO₂ and optionally reaction intermediates, flows upwards from below in the central pipe,
- the reaction mixture formed emerges from the central pipe in the upper part of the central pipe,
- the separation between melamine and off-gas takes place at the top of the reactor above the central pipe,
- one part of the melamine emerging above from the central pipe flows downwards in the annular space between the central pipe and reactor wall and the remaining part is expelled for further work-up,
- the off-gases are expelled continuously at the top of the reactor.

2. Process according to Claim 1, **characterized in that** the urea melt and optionally the NH₃ are introduced into the central pipe from below.

3. Process according to Claim 1, **characterized in that** the temperature of the melamine melt at the upper end of the central pipe is lower than at the lower end.

4. Process according to Claim 1, **characterized in that** the urea melt and optionally NH₃ introduced into the central pipe flow against a distribution plate in the lower part of the central pipe.

5. Process according to Claim 1, **characterized in that** the melamine melt circulating in the reactor enters into the central pipe through side openings arranged in the lower part of the central pipe, and flows upwards in the central pipe and downwards in the annular space between the central pipe and reactor wall.

6. Reactor for the production of melamine by pyrolysis of urea, consisting of a vertical reactor body having a central pipe, feed lines for urea melt and optionally NH₃ installed in the lower part of the reactor, drain lines for the melamine melt formed and for the off-gases consisting essentially of NH₃ and CO₂ installed in the upper part of the reactor, heating appliances and optionally measuring and control appliances, in particular for temperature, pressure, flow quantities and height level of the melamine melt, **characterized in that** one or more outlet openings for the supply of urea melt and optionally NH₃ are arranged in the lower part and within the central pipe.

7. Reactor according to Claim 6, **characterized in that** a distribution plate is installed in the lower part of the central pipe, above the supply pipes for urea and optionally NH₃.

8. Reactor according to Claim 7, **characterized in that** the distribution plate is provided with one or more openings in the direction of the central pipe for the passage of the urea melt and optionally NH₃.

9. Reactor according to Claim 7, **characterized in that** the distribution plate has the form of a flat plate, a cone, a pyramid or a half-dish.

10. Reactor according to Claim 6, **characterized in that** fittings for the comparative moderation of the flow and the comminution of the gas bubbles and for the improvement of the mixing are situated in the central pipe.

11. Reactor according to Claim 6, **characterized in that** a baffle and above it a calming zone are situated at the top of the reactor above the central pipe in the separation zone.

12. Reactor according to Claim 6, **characterized in that** the central pipe has side openings in its lower and/or upper part.

## Revendications

1. Procédé pour la préparation de mélamine par pyrolyse d'urée dans un réacteur haute pression présentant un tuyau central vertical avec formation d'une masse fondue de mélamine, **caractérisé**
- **en ce que** la masse fondue de mélamine en circulation dans le réacteur se mélange dans la partie inférieure du réacteur avec une masse fondue d'urée introduite à partir du bas dans le réacteur et le cas échéant avec du NH₃ introduit
- **en ce que** le mélange réactionnel formé, constitué essentiellement de mélamine, de NH₃, de CO₂ et le cas échéant de produits secondaires de réaction s'écoule de bas en haut dans le tuyau central
- **en ce que** dans la partie supérieure du tuyau central, le mélange réactionnel formé sort du tuyau central
- **en ce que** la séparation de la mélamine et des effluents gazeux se produit en tête du réacteur, au-dessus du tuyau central
- **en ce qu'**une partie de la mélamine qui sort du haut du tuyau central s'écoule vers le bas dans l'espace annulaire entre le tuyau central et la paroi du réacteur et le reste est évacué pour un traitement ultérieur,
- **en ce que** les effluents gazeux sont évacués en continu en tête du réacteur.

2. Procédé selon la revendication 1, **caractérisé en ce que** la masse fondue d'urée et le cas échéant le NH₃ sont introduits dans le tuyau central à partir du bas.

3. Procédé selon la revendication 1, **caractérisé en ce que** la température de la masse fondue de mélamine en l'extrémité supérieure du tuyau central est inférieure à celle en l'extrémité inférieure.

4. Procédé selon la revendication 1, **caractérisé en ce que** la masse fondue d'urée introduite dans le tuyau central et le cas échéant le NH₃ s'écoulent dans la partie inférieure du tuyau central contre une plaque de répartition.

5. Procédé selon la revendication 1, **caractérisé en ce que** la masse fondue de mélamine en circulation dans le réacteur entre dans le tuyau central via des ouvertures latérales disposées dans la partie inférieure du tuyau central, s'écoule vers le haut dans le tuyau central et vers le bas dans l'espace annulaire entre le tuyau central et la paroi du réacteur.

6. Réacteur pour la préparation de mélamine par pyrolyse d'urée, constitué d'un corps de réacteur vertical avec un tuyau central, des alimentations disposées dans la partie inférieure du réacteur pour la masse fondue d'urée et le cas échéant du NH₃, des conduites d'évacuation disposées dans la partie supérieure du réacteur pour la masse fondue formée de mélamine et pour les effluents gazeux essentiellement constitués de NH₃ et de CO₂, des dispositifs de chauffage et le cas échéant de dispositifs de mesure et de régulation, en particulier pour la température, la pression, les débits et la hauteur de remplissage de la masse fondue de mélamine, **caractérisé en ce qu'**une ou plusieurs ouvertures pour l'alimentation de la masse fondue d'urée et le cas échéant du NH₃ sont disposées dans la partie inférieure et à l'intérieur du tuyau central.

7. Réacteur selon la revendication 6, **caractérisé en ce qu'**une plaque de répartition est disposée dans la partie inférieure du tuyau central, au-dessus des alimentations pour l'urée et le cas échéant le NH₃.

8. Réacteur selon la revendication 7, **caractérisé en ce que** la plaque de répartition est pourvue d'une ou de plusieurs ouvertures vers le tuyau central pour le passage de la masse fondue d'urée et le cas échéant du NH₃.

9. Réacteur selon la revendication 7, **caractérisé en ce que** la plaque de répartition présente la forme d'une plaque plate, d'un cône, d'une pyramide ou d'une demi-coquille.

10. Réacteur selon la revendication 6, **caractérisé en ce que** le tuyau central comporte des chicanes pour uniformiser l'écoulement et diviser les bulles de gaz ainsi que pour améliorer le mélange.

11. Réacteur selon la revendication 6, **caractérisé en ce qu'**une plaque déflectrice est disposée en tête du réacteur, au-dessus du tuyau central, dans la zone de séparation et au-dessus de celle-ci se trouve une zone de stabilisation.

12. Réacteur selon la revendication 6, **caractérisé en ce que** le tuyau central présente des ouvertures latérales dans sa partie inférieure et/ou supérieure.
